# EUROPEAN PATENT APPLICATION

(11) **EP 3 950 922 A1**
(43) Date of publication of application: **09.02.2022**
(21) Application number: 20785177.5
(22) Date of filing: 16.01.2020
(51) Int. Cl.: C12M 1/34, C12N 5/0793, G01N 33/483

(54) **CELL EVALUATION DEVICE, METHOD FOR OPERATING CELL EVALUATION DEVICE, AND PROGRAM FOR OPERATING CELL EVALUATION DEVICE**

(30) Priority: 29.03.2019 JP 2019067744
(71) Applicant: FUJIFILM Corporation, Tokyo 106-8620 (JP)
(72) Inventor: MAEDA, Kiyohiro, Ashigarakami-gun, Kanagawa 258-8538 (JP)
(74) Representative: Klunker IP Patentanwälte PartG mbB
(86) International application number: PCT/JP2020/001264
(87) International publication number: WO 2020/202702

(57) **Abstract**

There is provided a cell evaluation device including an acquisition unit that acquires a cell image in which a nerve cell having a cell body, an axon, and a dendrite is shown; a first specifying unit that specifies the axon in the cell image; a second specifying unit that specifies spines formed on the dendrite in the cell image; a selection unit that selects a synapse-estimated spine, which is estimated to constitute a synapse with the axon and is close to the axon specified in the first specifying unit, from the spines specified in the second specifying unit; and a display control unit that performs control to display the synapse-estimated spine in a display form different from that of other spines in the cell image.

## Description

### BACKGROUND OF THE INVENTION

### 1. Field of the Invention

The techniques of the present disclosure relate to a cell evaluation device, an operation method for a cell evaluation device, and an operation program for a cell evaluation device.

### 2. Description of the Related Art

A nerve cell has a cell body, an axon, and a dendrite. The axon and the dendrite extend from the cell body and branch in some places during growth process. The axon plays a role in outputting a neurotransmitter to another nerve cell. On the contrary, the dendrite plays a role in receiving a neurotransmitter from another nerve cell.

The distal end of the axon is slightly bulged, and this bulged part is called the presynaptic terminal. In contrast, a plurality of spines, which are spinous protrusions, are formed on the surface of the dendrite. A part of the plurality of spines of a certain nerve cell and a presynaptic terminal of another nerve cell constitute a synapse for giving and taking a neurotransmitter.

JP2006-343853A describes a technique for specifying spines in a cell image in which a nerve cell is shown by subjecting the cell image in which a nerve cell is shown to the binarization processing, the boundary tracking processing, and the thinning processing in order.

### SUMMARY OF THE INVENTION

Neural transmission is the primary function of a nerve cell. For this reason, a synapse that is actually in charge of neural transmission is important as a structure for evaluating a nerve cell.

Although spines are specified in JP2006-343853A, not all the spines constitute synapses as described. As a result, nerve cells could not be sufficiently evaluated in JP2006-343853A.

An object of the technique of the present disclosure is to provide a cell evaluation device with which a nerve cell can be evaluated in more detail, an operation method for a cell evaluation device, and an operation program for a cell evaluation device.

In order to achieve the above object, the cell evaluation device of the present disclosure includes an acquisition unit that acquires a cell image in which a nerve cell having a cell body, an axon, and a dendrite is shown; a first specifying unit that specifies the axon in the cell image; a second specifying unit that specifies spines formed on the dendrite in the cell image; a selection unit that selects a synapse-estimated spine, which is estimated to constitute a synapse with the axon and is close to the axon specified in the first specifying unit, from the spines specified in the second specifying unit; and a display control unit that performs control to display the synapse-estimated spine in a display form different from that of other spines in the cell image.
it is preferable that the selection unit selects a spine that is present at a distance of less than 1 µm in terms of actual size from the axon specified in the first specifying unit, as the synapse-estimated spine.

It is preferable that an output control unit that performs control to output the number of synapse-estimated spines is provided. In this case, it is preferable that the output control unit performs control to output a proportion of the number of the synapse-estimated spines with respect to the total number of the spines specified in the second specifying unit.

It is preferable that a third specifying unit that specifies a dendrite in the cell image is provided and the output control unit performs control to output at least any one of the number of the synapse-estimated spines per unit length of the dendrite specified in the third specifying unit or the number of the synapse-estimated spines per unit area of the dendrite specified in the third specifying unit.

It is preferable that a fourth specifying unit that specifies, for every nerve cell, the axon and the dendrite that extend from the cell body is provided and the selection unit excludes axons and dendrites, which have been specified to extend from a cell body of the same nerve cell in the fourth specifying unit, from the selection target for the synapse-estimated spine.

It is preferable that the acquisition unit acquires a stained cell image in which a nerve cell in which at least any one of an axon or a dendrite is stained is shown, as a cell image.

The operation method for a cell evaluation device of the present disclosure includes an acquisition step of acquiring a cell image in which a nerve cell having a cell body, an axon, and a dendrite is shown; a first specification step of specifying the axon in the cell image; a second specification step of specifying spines formed on the dendrite in the cell image; a selection step of selecting a synapse-estimated spine, which is estimated to constitute a synapse with the axon and is close to the axon specified in the first specification step, from the spines specified in the second specification step; and a display control step of performing control to display the synapse-estimated spine in a display form different from that of other spines in the cell image.

The operation program for a cell evaluation device of the present disclosure causes a computer to function as an acquisition unit that acquires a cell image in which a nerve cell having a cell body, an axon, and a dendrite is shown; a first specifying unit that specifies the axon in the cell image; a second specifying unit that specifies spines formed on the dendrite in the cell image; a selection unit that selects a synapse-estimated spine, which is estimated to constitute a synapse with the axon and is close to the axon specified in the first specifying unit, from the spines specified in the second specifying unit; and a display control unit that performs control to display the synapse-estimated spine in a display form different from that of other spines in the cell image.

According to the technique of the present disclosure, it is possible to provide a cell evaluation device with which a nerve cell can be evaluated in more detail, an operation method for a cell evaluation device, and an operation program for a cell evaluation device.

### BRIEF DESCRIPTION OF THE DRAWINGS

Fig. 1 is a diagram illustrating a cell evaluation device and the like.
Fig. 2 is a diagram illustrating a structure of nerve cells.
Fig. 3 is a flowchart illustrating a flow of culturing nerve cells.
Fig. 4 is a block diagram illustrating a computer that constitutes the cell evaluation device.
Fig. 5 is a block diagram illustrating a processing unit of a CPU of the cell evaluation device.
Fig. 6 is a flowchart illustrating a flow of processing of specifying an axon, a dendrite, and a spine in a specifying unit.
Fig. 7 is a diagram illustrating an outline of a method of extracting a protrusive structure.
Fig. 8 is a diagram illustrating an outline of a method of determining a branch position of the protrusive structure.
Fig. 9 is a diagram illustrating morphological features of the axon and the dendrite.
Fig. 10 is a diagram illustrating a spine extraction procedure.
Fig. 11 is a diagram illustrating a specification result information.
Fig. 12 is a diagram illustrating selection conditions.
Fig. 13 is a diagram illustrating an example of the selection of a synapse-estimated spine.
Fig. 14 is a diagram illustrating the spine information.
Fig. 15 is a diagram illustrating a selection result display screen.
Fig. 16 is a flowchart illustrating a processing procedure of the cell evaluation device.
Fig. 17 is a block diagram illustrating a processing unit of a CPU of a cell evaluation device of the second embodiment.
Fig. 18 is a diagram illustrating a selection result display screen of the second embodiment.
Fig. 19 is a block diagram illustrating a processing unit of a CPU of a cell evaluation device of the third embodiment.
Fig. 20 is a flowchart illustrating a flow of culturing nerve cells according to the third embodiment.
Fig. 21 is a diagram illustrating an image set.
Fig. 22 is a flowchart illustrating a flow of processing of specifying, for every nerve cell, a protrusive structure that extends from a cell body in the specifying unit.
Fig. 23 is a diagram illustrating the processing of a step ST630, a step ST640, and a step ST650 in Fig. 22 in a case of K = 1.
Fig. 24 is a diagram illustrating the processing of a step ST630, a step ST640, and a step ST650 in Fig. 22 in a case of K = 2.
Fig. 25 is a diagram illustrating the processing of a step ST630, a step ST640, and a step ST650 in Fig. 22 in a case of K = 3.
Fig. 26 is a diagram illustrating regulations for determining the connectivity of the protrusive structure.
Fig. 27 is a diagram illustrating a state where a selection unit excludes axons and dendrites, which have been specified to extend from a cell body of the same nerve cell in the selection unit, from the selection target for the synapse-estimated spine.
Fig. 28 is a flowchart illustrating a flow of culturing nerve cells according to the fourth embodiment.
Fig. 29 is a diagram illustrating a state where an acquisition unit acquires a stained cell image in which a nerve cell in which at least any one of the axon or the dendrite is stained is shown.

### DESCRIPTION OF THE PREFERRED EMBODIMENTS

### [First embodiment]

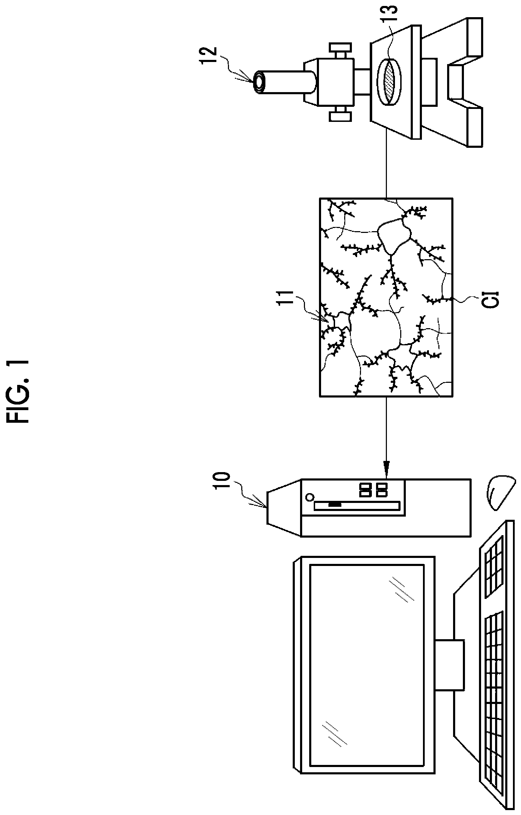

In Fig. 1, a cell evaluation device 10 is, for example, a device that evaluates a nerve cell 11 induced to differentiate from an induced pluripotent stem (iPS) cell and is, for example, a desktop personal computer. A cell image CI in which the nerve cell 11 is shown is input to the cell evaluation device 10. The cell image CI is taken by an operator with an imaging device 12 such as a digital phase-contrast microscope. A reference numeral 13 indicates a culture instrument such as a petri dish.

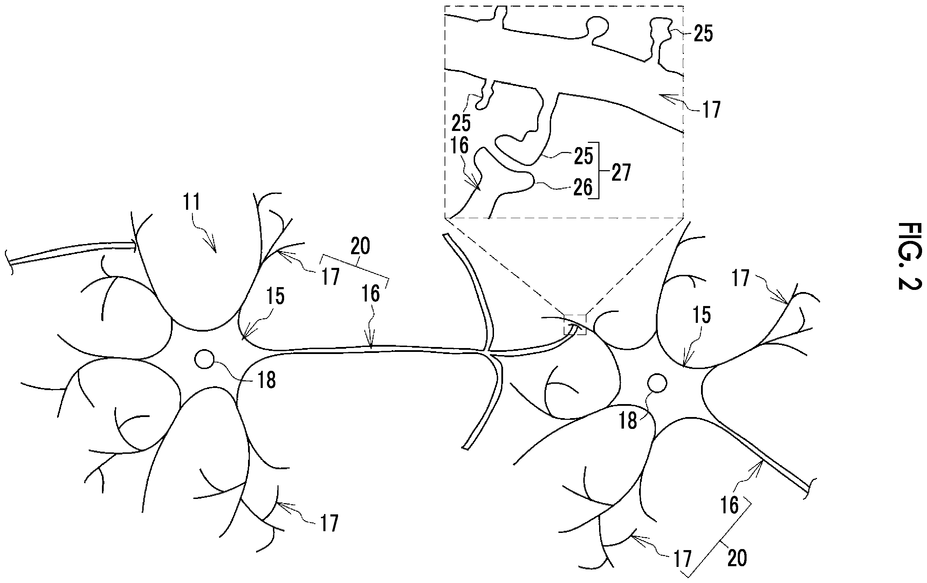

As illustrated in Fig. 2, the nerve cell 11 has a cell body 15, an axon 16, and a dendrite 17. The cell body 15 has a nucleus 18 and forms the center of the nerve cell 11. The axon 16 extends from the cell body 15 and plays a role in outputting a neurotransmitter to another nerve cell 11. Basically, only one axon 16 extends from one cell body 15; however, a branch called an axon collateral is formed during the growth process. The dendrite 17 also extends from the cell body 15. However, contrary to the axon 16, the dendrite 17 plays a role in receiving a neurotransmitter from another nerve cell 11. Unlike the axon 16, a plurality of dendrites 17 may extend from one cell body 15. In addition, the dendrite 17 spreads while literally branching into a dendritic shape during the growth process. Hereinafter, the axon 16 and the dendrite 17 are collectively written as a protrusive structure 20.

As illustrated inside the broken line, a plurality of spines 25, which are spinous protrusions, are formed on the surface of the dendrite 17. A part of the plurality of spines 25 and a presynaptic terminal 26 of another nerve cell 11 constitute a synapse 27 for giving and taking a neurotransmitter. The presynaptic terminal 26 is a bulged part at the distal end of the axon 16.

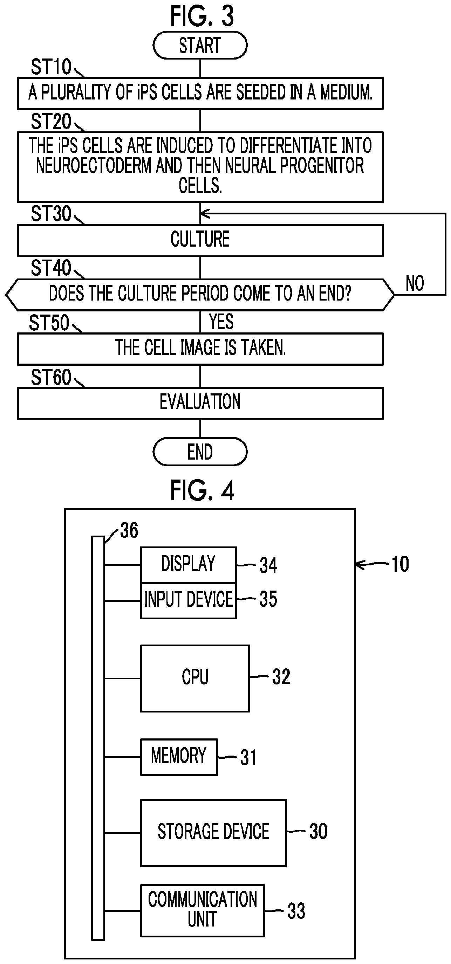

Fig. 3 is a flowchart illustrating a flow of culturing the nerve cell 11. First, an operator seeds a plurality of iPS cells in a medium of a culture instrument 13 (a step ST10). Then, the iPS cells are induced to differentiate into neuroectoderm and then neural progenitor cells in order (a step ST20). As a result, the culture of the nerve cell 11 is started (a step ST30).

After the preset culture period comes to an end (YES in a step ST40), the operator takes the cell image CI using the imaging device 12 (a step ST50). The operator evaluates the nerve cell 11 using the cell evaluation device 10 based on the taken cell image CI (a step ST60).

In Fig. 4, a computer constituting the cell evaluation device 10 includes a storage device 30, a memory 31, a central processing unit (CPU) 32, a communication unit 33, a display 34, and an input device 35. These components are connected to each other through a busline 36.

The storage device 30 is a hard disk drive that is built in the computer that constitutes the cell evaluation device 10 or is connected to the computer through a cable or a network. Alternatively, the storage device 30 is a disk array in which a plurality of hard disk drives are connectively mounted. The storage device 30 stores a control program such as an operating system, various application programs, various types of data associated with these programs, and the like.

The memory 31 is a work memory that is used in a case where the CPU 32 executes processing. The CPU 32 loads the program stored in the storage device 30 into the memory 31 and executes processing according to the program, thereby comprehensively controlling each of the units of the computer.

The communication unit 33 is a network interface that controls the transmission of various information via a network such as a local area network (LAN) or a wide area network (WAN). The display 34 displays various screens. The computer that constitutes the cell evaluation device 10 receives an input of an operation command from the input device 35, through the various screens. The input device 35 is a keyboard, a mouse, a touch panel, or the like.

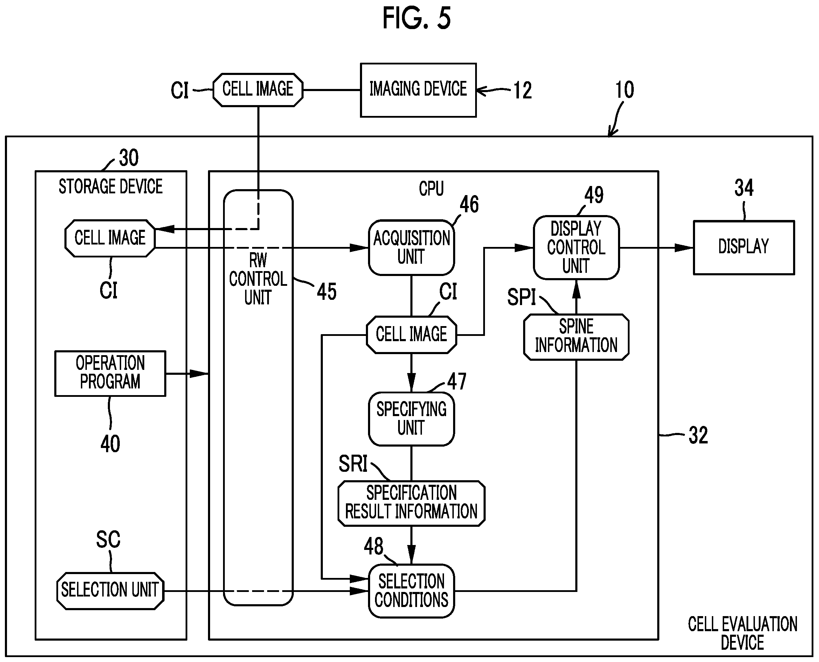

In Fig. 5, an operation program 40 is stored in the storage device 30 of the cell evaluation device 10. The operation program 40 is an application program for making a computer function as the cell evaluation device 10. That is, the operation program 40 is an example of the "operation program for the cell evaluation device" according to the technique of the present disclosure. The storage device 30 also stores the cell image CI and selection conditions SC.

In a case where the operation program 40 is activated, the CPU 32 of the computer constituting the cell evaluation device 10 cooperates with the memory 31 and the like to function as a read and write (hereinafter, abbreviated as RW) control unit 45, an acquisition unit 46, a specifying unit 47, a selection unit 48, and a display control unit 49.

The RW control unit 45 controls the reading-out of various data in the storage device 30 and the storage of various data in the storage device 30. The RW control unit 45 stores the cell image CI from the imaging device 12 in the storage device 30. The RW control unit 45 reads out the cell image CI from the storage device 30 and outputs the read-out cell image CI to the acquisition unit 46. Further, the RW control unit 45 reads out the selection conditions SC from the storage device 30 and outputs the read-out the selection conditions SC to the selection unit 48.

The acquisition unit 46 acquires the cell image set CI from the RW control unit 45. The acquisition unit 46 outputs the cell image CI to the specifying unit 47, the selection unit 48, and the display control unit 49.

The specifying unit 47 specifies the axon 16, the dendrite 17, and the spine 25 in the cell image CI. That is, the specifying unit 47 is an example of the "first specifying unit", the "second specifying unit", and the "third specifying unit" according to the technique of the present disclosure. The specifying unit 47 creates a specification result information SRI that indicates the specification results of the axon 16, the dendrite 17, and the spine 25. The specifying unit 47 outputs the specification result information SRI to the selection unit 48.

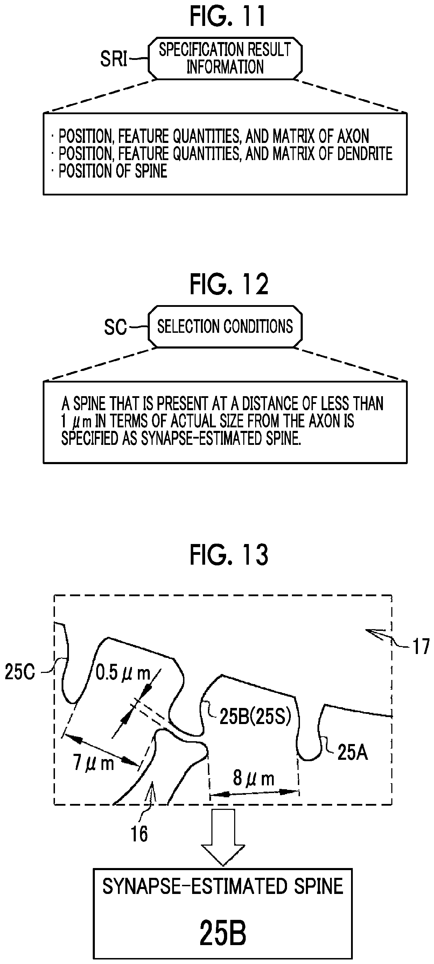

The selection unit 48 selects a synapse-estimated spine 25S (see Fig. 13) from the spines 25 specified in the specifying unit 47, based on the specification result information SRI and the selection conditions SC. The synapse-estimated spine 25S is the spine 25 that is estimated to constitute the synapse 27 with the axon 16, among the spines 25, and is the spine 25 that is close to the axon 16 specified in the specifying unit 47. The selection unit 48 creates a spine information SPI including information on the selected synapse-estimated spine 25S. The selection unit 48 outputs the spine information SPI to the display control unit 49.

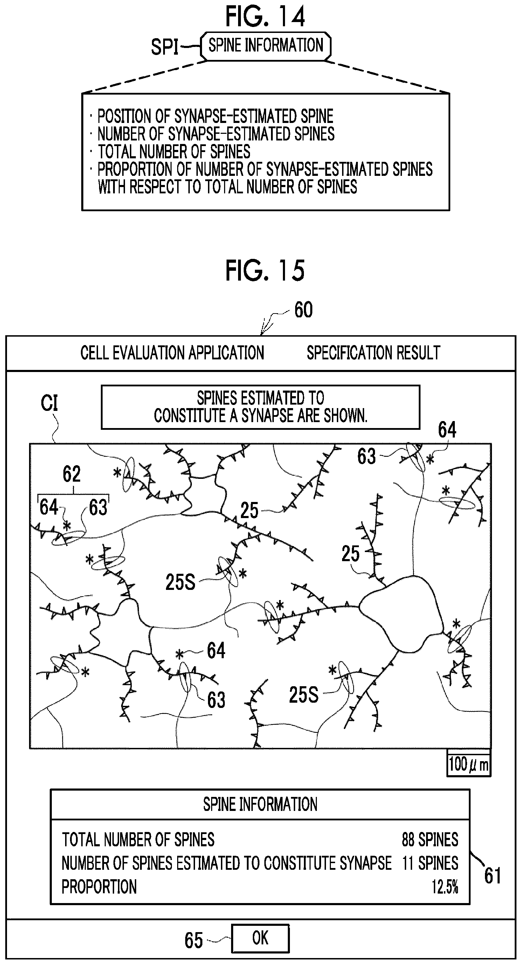

The display control unit 49 performs control to display various screens on the display 34. For example, the display control unit 49 performs control to display a selection result display screen 60 (see Fig. 15) or the like, which shows the selection result of the synapse-estimated spine 25S by the selection unit 48, on the display 34.

Further, the display control unit 49 performs control to display displaying an instruction reception screen (not illustrated in the drawing) for receiving an instruction from the operator for causing the selection unit 48 to select the synapse-estimated spine 25S, on the display 34. The cell image CI and an instruction button for instructing the selection of the synapse-estimated spine 25S are provided on the instruction reception screen. In a case where the instruction button is selected by the operator on this instruction reception screen, the processing described below is executed.

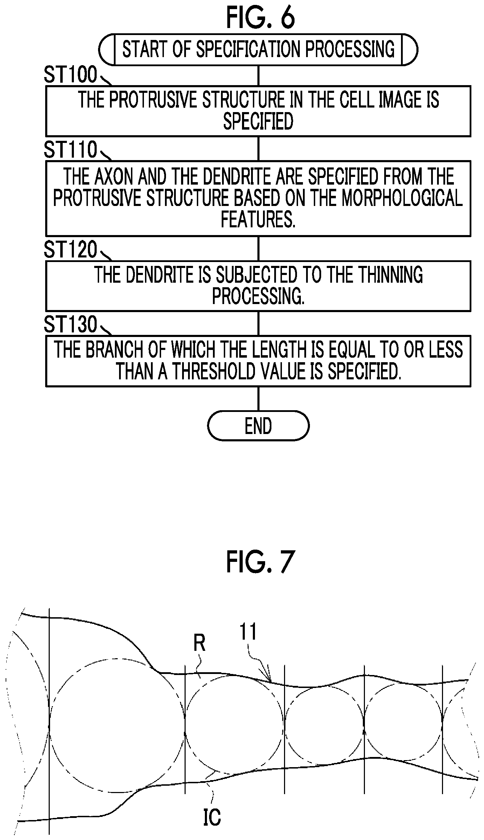

Fig. 6 is a flowchart illustrating a flow of processing of specifying the axon 16, the dendrite 17, and the spine 25 in the specifying unit 47. First, the specifying unit 47 specifies a plurality of protrusive structures 20 in the cell image CI (a step ST100).

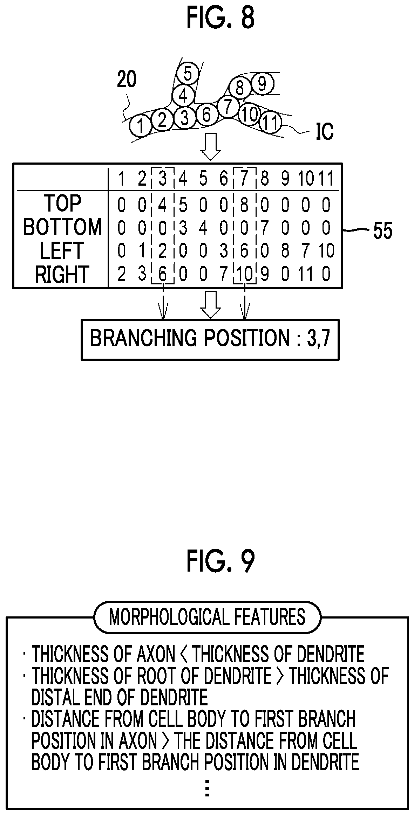

The specifying unit 47 specifies the axon 16 and the dendrite 17 from the plurality of protrusive structures 20 specified in the step ST100 based on the morphological features (see Fig. 9) (a step ST110). In other words, the specifying unit 47 classifies the plurality of protrusive structures 20 specified in the step ST100 into the axon 16 and the dendrite 17. The step ST110 is an example of the "first specification step" according to the technique of the present disclosure.

The specifying unit 47 subjects the dendrite 17 specified in the step ST110 to the thinning processing (a step ST120). Then, the dendrite 17 that has been subjected to the thinning processing is scanned. Among the branches recognized in the scanning process, the specifying unit 47 specifies a branch of which the length is equal to or less than a threshold value as the spine 25 (a step ST130). The threshold value is set to, for example, 3 µm in terms of actual size. The step ST130 is an example of the "second specification step" according to the technique of the present disclosure. The method of specifying the axon 16, the dendrite 17, and the spine 25 is not limited to the method described above. For example, the spine 25 may be specified by using the method described in JP2006-343853A.

The specification of the protrusive structure 20, which is represented in the step ST100 of Fig. 6, is carried out, for example, by using the technique described in JP2009-063509A. In the technique described in JP2009-063509A, the outline of which is illustrated in Fig. 7, first, the cell image CI is subjected to the binarization processing, thereby being divided into a region constituting the nerve cell 11 and a region not constituting the nerve cell 11. Then, inscribed circles IC are provided so that they are not overlapped in the region constituting the nerve cell 11. Next, the region constituting the nerve cell 11 is divided into a plurality of small regions R including each of the inscribed circles IC. Subsequently, for each small region R, feature quantities such as the center point of the inscribed circle IC, the radius of the inscribed circle IC, and the average value of the pixel values are calculated. According to the center point of the inscribed circle IC, the shape of the nerve cell 11 can be traced almost accurately. The specifying unit 47 specifies, for example, a small region R in which the radius of the inscribed circle IC is equal to or shorter than a threshold value as the protrusive structure 20. The method of specifying the protrusive structure 20 is not limited to the method described in JP2009-063509A.

Further, the specifying unit 47 derives a matrix 55 illustrated in Fig. 8 by using the technique described in JP2009-063509A. The matrix 55 represents the connection relationship of the inscribed circles IC provided in the protrusive structure 20. In the matrix 55, the number of each of the inscribed circles IC of the protrusive structure 20 is arranged in the first row. Then, under the number of each of the inscribed circles IC, the numbers of the inscribed circles IC which are connected to the top, the bottom, the left, and the right, respectively, are arranged. For example, with respect to the inscribed circle IC of number 3, the inscribed circles IC of number 2, number 4, and number 6 are connected to the left, top, and right, respectively, and thus 4, 0 (which means the connection is not present), 2, and 6 are lined up below the number 3. According to such a matrix 55, it is possible to determine the branch position of the protrusive structure 20 and eventually the branch positions of the axon 16 and the dendrite 17. The method of determining the branch position is not limited to the method described in JP2009-063509A.

The content of the morphological features referred to in a case of specifying the axon 16 and the dendrite 17, which is represented in the step ST110 of Fig. 6, is for example, as illustrated in Fig. 9. That is, the content thereof includes that the axon 16 is thinner than the dendrite 17 (the thickness of the axon 16 < thickness of the dendrite 17). In addition, the content thereof includes that in the dendrite 17, the distal end is thinner than the root (in the vicinity of the cell body 15) (the thickness of the root of the dendrite 17 > the thickness of the distal end of the dendrite 17). Further, the content thereof includes that the axon 16 has a longer distance than the dendrite 17 in terms of the distance from the cell body 15 to the first branch position (the distance from the cell body 15 to the first branch position in the axon 16 > the distance from the cell body 15 to the first branch position in the dendrite 17). Using these morphological features, the feature quantities for every small region R, and the branch position known from the matrix 55 as clues, the specifying unit 47 separates the protrusive structure 20 into the axon 16 and the dendrite 17.

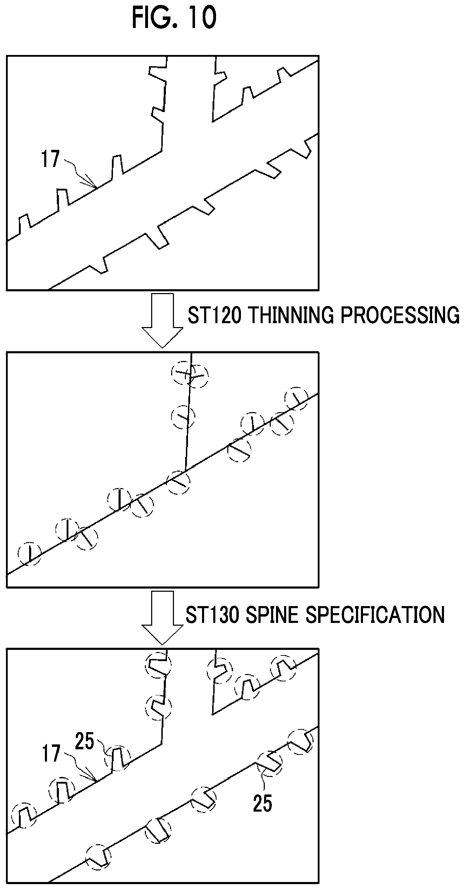

Fig. 10 shows a state where the dendrite 17 is subjected to the thinning processing, which is represented in the step ST120 of Fig. 6, and a state where a branch of which the length is equal to or less than a threshold value is specified as the spine 25, which is represented in the step ST130 of Fig. 6. In a case of being subjected to such processing, the spine 25 is specified by the specifying unit 47.

As illustrated in Fig. 11, the specification result information SRI includes the position of the axon 16, the feature quantities for every small region R of the axon 16, the matrix 55 of the axon 16, the position of the dendrite 17, the feature quantities for every small region R of the dendrite 17, the matrix 55 of the dendrite 17, and the position of the spine 25. The position of the axon 16, the position of the dendrite 17, and the position of the spine 25 are specifically represented by the coordinates of the pixels of the cell image CI.

As illustrated in Fig. 12, the content of the selection conditions SC is that the spine 25 present at a distance of less than 1 µm in terms of actual size from the axon 16 is selected as the synapse-estimated spine 25S. As a result, for example, as illustrated in Fig. 13, in a case where there are a spine 25A present at a distance of 8 µm from the axon 16, a spine 25B present at a distance of 0.5 µm from the axon 16, and a spine 25C present at a distance of 7 µm from the axon 16, the selection unit 48 selects the spine 25B present at a distance of less than 1 µm from the axon 16 as the synapse-estimated spine 25S.

As illustrated in Fig. 14, the spine information SPI includes the position and the number of synapse-estimated spines 25S selected in the selection unit 48, the total number of spines 25 specified in the specifying unit 47, and the proportion of the number of synapse-estimated spines 25S with respect to the total number of spines 25.

In Fig. 15, the cell image CI and a display frame 61 of the spine information SPI are displayed on the selection result display screen 60 that is displayed on the display 34 based on the spine information SPI under the control of the display control unit 49. A marker 62 is attached to the synapse-estimated spine 25S in the cell image CI. The marker 62 is composed of an elliptical frame 63 that surrounds the synapse-estimated spine 25S and an asterisk 64 that points to and indicates the frame 63. That is, the display control unit 49 performs control to display the synapse-estimated spine 25S in a display form different from that of other spines 25 in the cell image CI.

A list of the total number of spines 25, the number of synapse-estimated spines 25S (written as "the number of spines estimated to constitute a synapse" in Fig. 15), and the proportion of the number of synapse-estimated spines 25S (simply written as the "proportion" in Fig. 15) with respect to the total number of spines 25, among the spine information SPI, is displayed in the display frame 61. That is, the display control unit 49 is an example of the "output control unit" according to the technique of the present disclosure. In a case where an OK button 65 is selected, the display control unit 49 turns off the display of the selection result display screen 60.

As the method of displaying the synapse-estimated spine 25S in a display form different from that of other spines 25, a method of coloring the synapse-estimated spine 25S with a color different from that of other spines 25, such as red, may be adopted. Further, a method in which a state where the synapse-estimated spine 25S is colored and a state where the synapse-estimated spine 25S is not colored are alternately repeated at intervals of several seconds may be adopted.

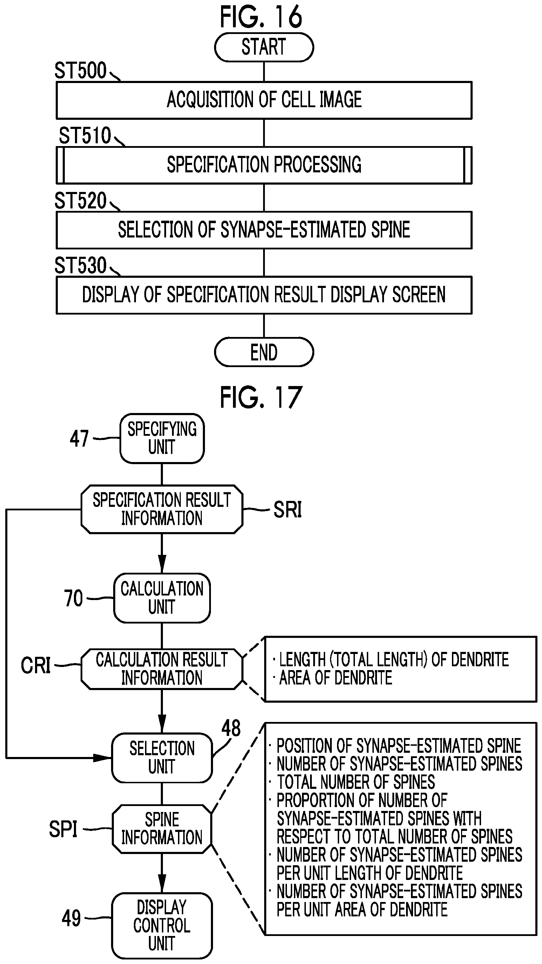

Next, an operation based on the above configuration will be described with reference to the flowchart of Fig. 16. First, in a case where the operation program 40 is activated in the cell evaluation device 10, as illustrated in Fig. 5, the CPU 32 of the cell evaluation device 10 is allowed to function as the RW control unit 45, the acquisition unit 46, the specifying unit 47, the selection unit 48, and the display control unit 49.

In the cell evaluation device 10, the cell image CI is read out from the storage device 30 by the RW control unit 45 and is output to the acquisition unit 46. As a result, the cell image CI is acquired by the acquisition unit 46 (a step ST500). The cell image CI is output from the acquisition unit 46 to the specifying unit 47, the selection unit 48, and the display control unit 49. The step ST500 is an example of the "acquisition step" according to the technique of the present disclosure.

The specifying unit 47 executes the specification processing illustrated in Fig. 6 and the like to specify the axon 16, the dendrite 17, and the spine 25 in the cell image CI (a step ST510). The specification processing of the step ST510 is the processing that includes the step ST110 which is an example of the "first specification step" and the step ST130 which is an example of the "second specification step", which are illustrated in Fig. 6.

In the specifying unit 47, the specification result information SRI illustrated in Fig. 11 is created. The specification result information SRI is output from the specifying unit 47 to the selection unit 48.

As illustrated in Fig. 13, the selection unit 48 selects the synapse-estimated spine 25S from the spines 25 specified in the specifying unit 47 (a step ST520). The selection unit 48 creates the spine information SPI illustrated in Fig. 14. The spine information SPI is output from the selection unit 48 to the display control unit 49. The step ST520 is an example of the "selection step" according to the technique of the present disclosure.

As illustrated in Fig. 15, the display control unit 49 attaches the marker 62 to the synapse-estimated spine 25S and displays the selection result display screen 60 in which the synapse-estimated spine 25S has a display form different from that of other spines 25, on the display 34 (a step ST530). The operator confirms the synapse-estimated spine 25S through the selection result display screen 60 and evaluates the growth state of the nerve cell 11. The step ST530 is an example of a "display control step" according to the technique of the present disclosure.

As described above, in the cell evaluation device 10, the acquisition unit 46 acquires the cell image CI in which the nerve cell 11 is shown. The specifying unit 47 specifies the axon 16 and the spine 25 in the cell image CI. Next, the selection unit 48 selects the synapse-estimated spine 25S, which is estimated to constitute the axon 16 and the synapse 27, from the specified spine 25. Then, the display control unit 49 performs control to display of the synapse-estimated spine 25S in a display form different from that of other spines 25 in the cell image CI. That is, the cell evaluation device 10 not only specifies the spine 25 but also selects the synapse-estimated spine 25S among the spines 25, and displays it. As a result, it is possible to evaluate the nerve cells 11 in more detail.

As illustrated in Fig. 12 and Fig. 13, the selection unit 48 selects the spine 25 present at a distance of less than 1 µm in terms of actual size from the axon 16 specified in the specifying unit 47, as the synapse-estimated spine 25S. The distance between the spine 25 and the presynaptic terminal 26 in the actual synapse 27 is several tens of nanometers. Therefore, in a case where the selection conditions SC are set to "1 µm in terms of actual size", it is possible to thoroughly select the spine 25 that actually constitutes the synapse 27 as the synapse-estimated spine 25S.

As illustrated in the display frame 61 of Fig. 15, the display control unit 49 performs control to display of the number of synapse-estimated spines 25S and the proportion of the number of synapse-estimated spines 25S with respect to the total number of spines 25. The number of synapse-estimated spines 25S and the proportion of the number of synapse-estimated spines 25S with respect to the total number of spines 25 are important index values for knowing the growth state of the nerve cell 11. Specifically, it can be said that the larger the number of synapse-estimated spines 25 S and the larger the proportion of the number of synapse-estimated spines 25S with respect to the total number of spines 25, the better the nerve cell 11 has grown. As a result, the growth state of the nerve cell 11 can be quantitatively evaluated.

The output form of the number of synapse-estimated spines 25S and the proportion of the number of synapse-estimated spines 25S with respect to the total number of spines 25 is not limited to the selection result display screen 60 of Fig. 15. A form of printing out on a paper medium or a form of outputting as a data file may be adopted instead of or in addition to the selection result display screen 60.

### [Second embodiment]

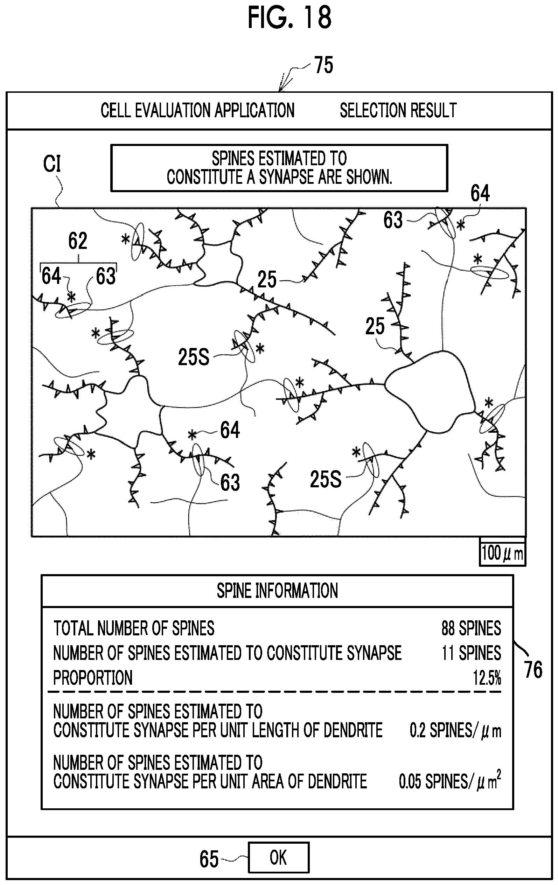

In the second embodiment illustrated in Fig. 17 and 18, the control to output the number of synapse-estimated spines 25S per unit length of the dendrite 17 and the number of synapse-estimated spines 25S per unit area of the dendrite 17 is performed.

In Fig. 17, the CPU of the cell evaluation device of the second embodiment functions as a calculation unit 70 in addition to each of the processing units 45 to 49 (the RW control unit 45 and the acquisition unit 46 are not illustrated in Fig. 17) of the first embodiment. As described above, the specifying unit 47 is an example of the "third specifying unit" that specifies the dendrite 17 in the cell image CI. The specifying unit 47 outputs the specification result information SRI including the position, the feature quantities, and the matrix 55 of the dendrite 17, which is illustrated in Fig. 11, to the calculation unit 70. The calculation unit 70 calculates the length (the total length) and the area of the dendrite 17 based on the position, the feature quantities, and the matrix 55 of the dendrite 17, which are included in the specification result information SRI. The calculation unit 70 calculates the length of the dendrite 17, for example, by adding the diameters of the inscribed circles IC. In addition, the calculation unit 70 determines the area of the dendrite 17, for example, by counting the number of pixels constituting the dendrite 17. The calculation unit 70 outputs the calculation result information CRI including the length and the area of the dendrite 17 to the selection unit 48. As illustrated in Fig. 10, the length of the dendrite 17 may be calculated by subjecting the dendrite 17 to the thinning processing and then counting the number of pixels constituting the thinned dendrite 17.

In addition to the index values such as the number of synapse-estimated spines 25S described in the first embodiment, the selection unit 48 can incorporate the number of synapse-estimated spines 25S per unit length of the dendrite 17 and the number of synapse-estimated spines 25S per unit area of the dendrite 17 in the spine information SPI. The number of synapse-estimated spines 25S per unit length of the dendrite 17 is determined by dividing the number of synapse-estimated spines 25S by the length of the dendrite 17. In the same manner, the number of synapse-estimated spines 25S per unit area of the dendrite 17 is determined by dividing the number of synapse-estimated spines 25S by the area of the dendrite 17.

The display control unit 49 performs control to display a selection result display screen 75 illustrated in Fig. 18 on the display 34. The selection result display screen 75 is basically the same as the selection result display screen 60 of the first embodiment illustrated in Fig. 15; however, the displayed content of the spine information SPI in the display frame 76 is different from that of the selection result display screen 60. That is, in addition to the number of synapse-estimated spines 25S of the first embodiment, a list of the number of synapse-estimated spines 25S per unit length of the dendrite 17 (written as "the number of spines estimated to constitute a synapse per unit length of the dendrite" in Fig. 18) and the number of synapse-estimated spines 25S per unit area of the dendrite 17 (written as "the number of spines estimated to constitute a synapse per unit area of dendrites" in Fig. 18) is displayed in the display frame 76. As a result, in the second embodiment as well, the display control unit 49 is an example of the "output control unit" according to the technique of the present disclosure.

In this manner, in the second embodiment, the specifying unit 47 specifies the dendrite 17 in the cell image CI. Next, the calculation unit 70 calculates the length and the area of the dendrite 17. Then, the display control unit 49 outputs the number of synapse-estimated spines 25S per unit length of the dendrite 17 and the number of synapse-estimated spines 25S per unit area of the dendrite 17 through the selection result display screen 75. The number of synapse-estimated spines 25S per unit length of the dendrite 17 and the number of synapse-estimated spines 25S per unit area of the dendrite 17 are important index values for knowing the growth state of the nerve cell 11, similarly to the number of synapse-estimated spines 25S and the like. Specifically, it can be said that the larger the number of synapse-estimated spines 25S per unit length of dendrite 17 and the larger the number of synapse-estimated spines 25S per unit area of the dendrite 17, the better the nerve cell 11 has grown. As a result, the growth state of the nerve cell 11 can be quantitatively evaluated.

The index value to be output may be at least any one of the number of synapse-estimated spines 25S per unit length of the dendrite 17 and the number of synapse-estimated spines 25S per unit area of the dendrite 17. Further, the output form of the number of synapse-estimated spines 25S per unit length of the dendrite 17 and the number of synapse-estimated spines 25S per unit area of the dendrite 17 is not limited to the selection result display screen 75 of Fig. 18, similarly to the second embodiment. A form of printing out on a paper medium or a form of outputting as a data file may be adopted instead of or in addition to the selection result display screen 75.

### [Third embodiment]

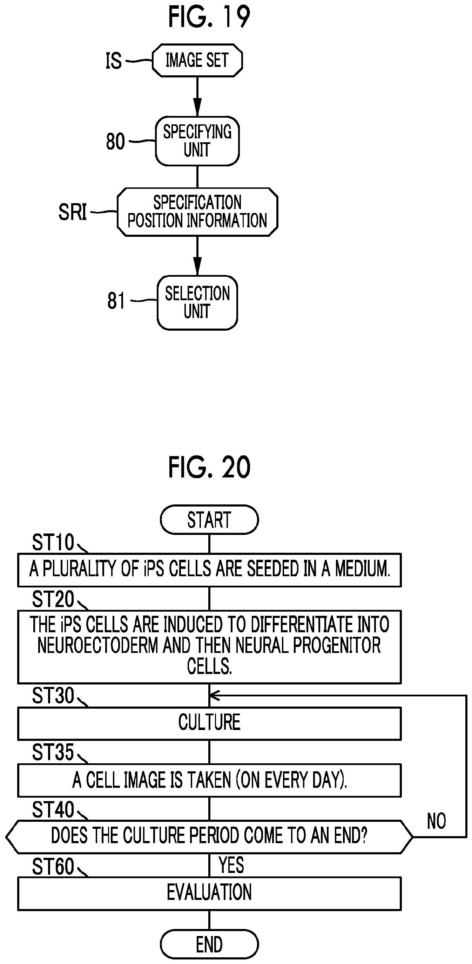

In the third embodiment illustrated in Fig. 19 to Fig. 27, the axon 16 and the dendrite 17 that extend from the cell body 15 are specified for every nerve cell 11. Then, the axon 16 and the dendrite 17, which have been specified to extend from the cell body 15 of the same nerve cell 11, are excluded from the selection target for the synapse-estimated spine 25S.

In Fig. 19, the specifying unit 80 of the cell evaluation device of the third embodiment has a function of specifying the axon 16, the dendrite 17, and the spine 25, which is the function that the specifying unit 47 of the first embodiment has. In addition, the specifying unit 80 has a function of specifying, for every nerve cell 11, the protrusive structure 20 (the axon 16 and the dendrite 17) that extends from the cell body 15. That is, the specifying unit 80 is an example of the "fourth specifying unit" according to the technique of the present disclosure.

The image set IS is input to the specifying unit 80. The specifying unit 80 captures the growth process of the protrusive structure 20 based on the image set IS to specify the protrusive structure 20 that extends from the cell body 15 of each of the nerve cells 11 in the cell image CI of the selection target for the synapse-estimated spine 25S. The specifying unit 80 creates the specification result information SRI including the specification result of the protrusive structure 20 for every nerve cell 11 and outputs the specification result information SRI to the selection unit 81.

As illustrated in Fig. 20, in the present embodiment, the operator takes the cell image CI during culture using the imaging device 12 at regular imaging intervals, for example, every day (a step ST35). At this time, the operator sets the culture instrument 13 in the imaging device 12 so that the imaging position is the same every time, and then takes the cell image CI.

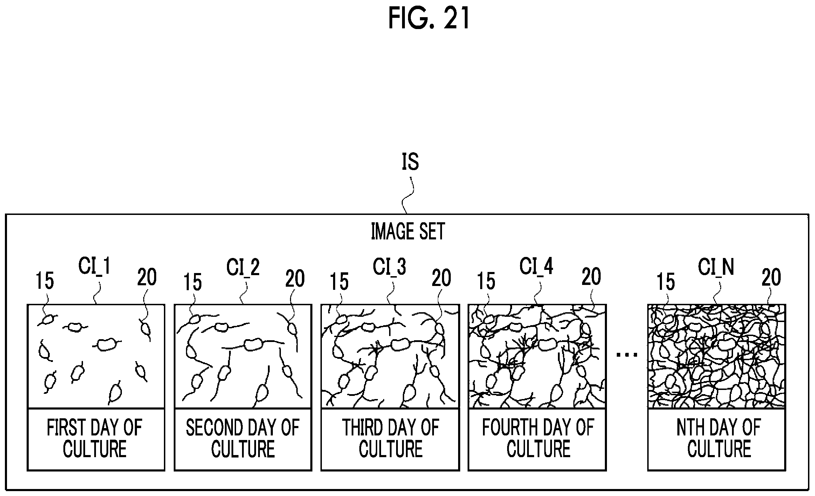

As illustrated in Fig. 21, the image set IS is a collection of a plurality of cell images CI in which the nerve cells 11 are imaged in time series during culture. In the case of the present example, since the cell image CI is taken every day, the image set IS is composed of a cell image CI_1 on the first day of culture (the start day of culture), a cell image CI_2 on the second day of culture, a cell image CI_3 on the third day of culture, a cell image CI_4 on the fourth day of culture, ..., and a cell image CI_N on the Nth day of culture (the last day of culture). The cell image CI_N on the Nth day of culture is the cell image CI of the selection target for the synapse-estimated spine 25S. The first day of culture is the day when the iPS cells are induced to differentiate into neural progenitor cells.

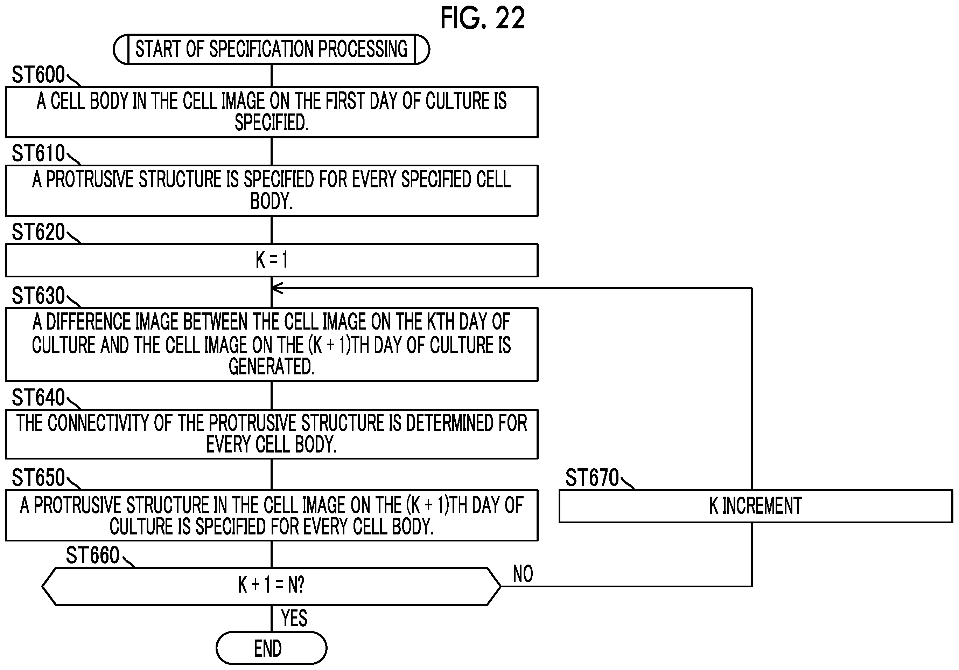

Fig. 22 is a flowchart illustrating a flow of processing of specifying, for every nerve cell 11, a protrusive structure 20 that extends from a cell body 15 in the specifying unit 80. First, the specifying unit 80 specifies the cell body 15 in the cell image CI_1 on the first day of culture (a step ST600). The specifying unit 80 specifies, for example, a small region R in which the radius of the inscribed circle IC is larger than a threshold value as the cell body 15. Next, the specifying unit 80 specifies the protrusive structure 20 for every cell body 15 specified in the step ST600 (a step ST610).

The specifying unit 80 generates a difference image DI_K, K + 1 between a cell image CI_K on the Kth day of culture and a cell image CI_K + 1 on the (K + 1)th day of culture (a step ST630). Then, the connectivity between a protrusive structure 20_K, K + 1 which is shown in the difference image DI_K, K + 1 and a protrusive structure 20_K which is shown in the cell image CI_K on the Kth day of culture is determined for every cell body 15 (a step ST640). The specifying unit 80 specifies a protrusive structure of interest 20_K + 1 for every cell body 15 in the cell image CI_K + 1 on the (K + 1)th day of culture based on the determination result of the connectivity in the step ST640 (a step ST650).

More specifically, first, the specifying unit 80 carries out processing of the step ST630, the step ST640, and the step ST650 with K = 1 (a step ST620). Then, the specifying unit 80 increments K (NO in a step ST660, and a step ST670) until K + 1 = N, and repeats the processing of the step ST630, the step ST640, and step the ST650.

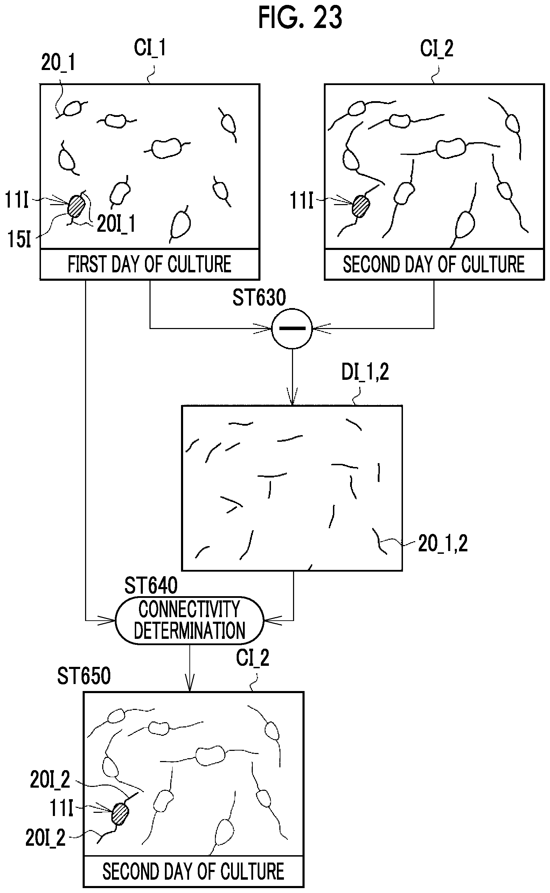

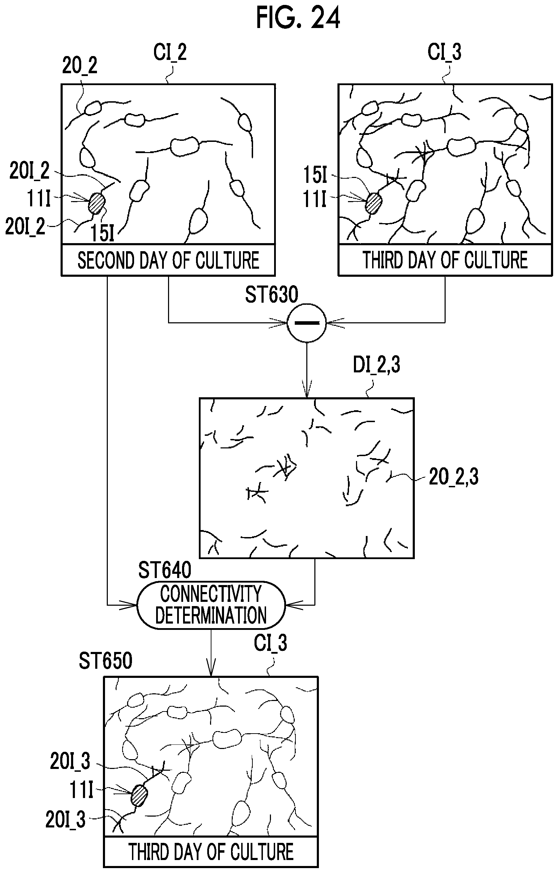

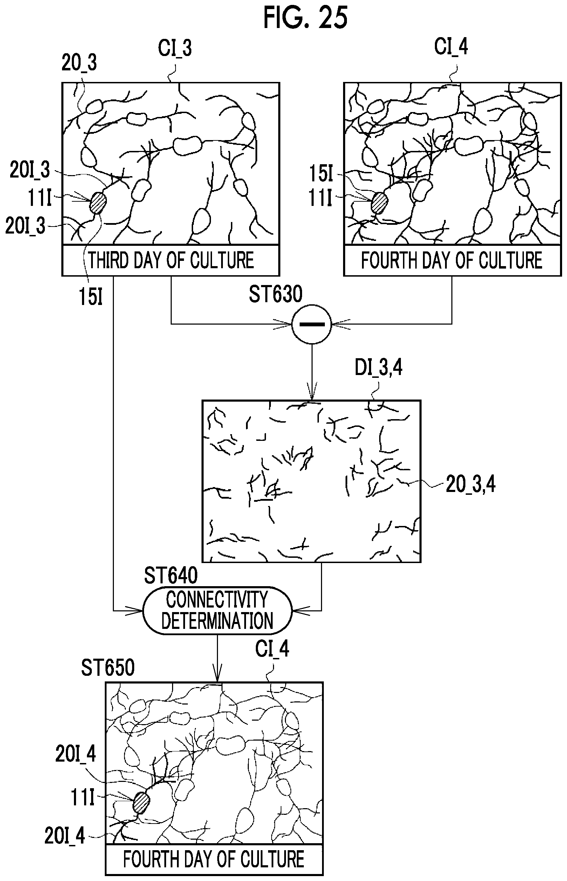

Fig. 23 to Fig. 25 are diagrams illustrating processing of the step ST630, the step ST640, and the step ST650 in Fig. 22. A case of K = 1 is illustrated in Fig. 23, a case of K = 2 is illustrated in Fig. 24, and a case of K = 3 is illustrated in Fig. 25. In Fig. 23 to Fig. 25, the cell body 151 and the protrusive structure 201 of one nerve cell 11I are displayed separately from others in order to assist understanding.

As illustrated in Fig. 23, the specifying unit 80 generates a difference image DI_1, 2 between the cell image CI_1 on the first day of culture and the cell image CI_2 on the second day of culture. Then, the connectivity between a protrusive structure 20_1, 2 which is shown in the difference image DI_1, 2 and a protrusive structure 20_1 which is shown in the cell image CI_1 on the first day of culture is determined. Based on the determination result of this connectivity, the specifying unit 80 specifies the protrusive structure 20_2 for every cell body 15 in the cell image CI_2 on the second day of culture.

In the same manner, as illustrated in Fig. 24, the specifying unit 80 generates a difference image DI_2, 3 between the cell image CI_2 on the second day of culture and the cell image CI_3 on the third day of culture. Then, the connectivity between a protrusive structure 20_2, 3 which is shown in the difference image DI_2, 3 and a protrusive structure 20_2 which is shown in the cell image CI_2 on the second day of culture is determined. Based on the determination result of this connectivity, the specifying unit 80 specifies the protrusive structure 20_3 for every cell body 15 in the cell image CI_3 on the third day of culture.

Further, as illustrated in Fig. 25, the specifying unit 80 generates a difference image DI_3, 4 between the cell image CI_3 on the third day of culture and the cell image CI_4 on the fourth day of culture. Then, the connectivity between a protrusive structure 20_3, 4 which is shown in the difference image DI_3, 4 and a protrusive structure 20_3 which is shown in the cell image CI_3 on the third day of culture is determined. Based on the determination result of this connectivity, the specifying unit 80 specifies the protrusive structure 20_4 for every cell body 15 in the cell image CI_4 on the fourth day of culture. By repeating such processing until K + 1 = N is satisfied, the specifying unit 80 finally specifies, for every cell body 15, the protrusive structure 20_N in the cell image CI_N on the Nth day of culture.

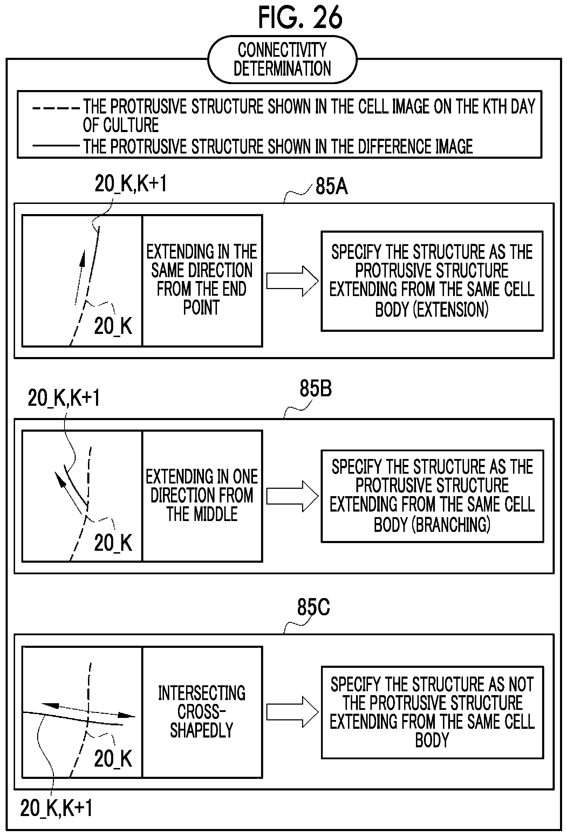

The determination of the connectivity in the step ST640 is carried out, for example, according to a first regulation 85A, a second regulation 85B, and a third regulation 85C, which are illustrated in Fig. 26. The content of the first regulation 85A is that in a case where the protrusive structure 20_K, K + 1 which is shown in the difference image DI_K, K + 1, where the protrusive structure 20_K, K + 1 is indicated by the solid line, extends in the same direction from the end point of the protrusive structure 20_K which is shown in the cell image CI_K on the Kth day, where the protrusive structure 20_K is indicated by the broken line, it is determined that the protrusive structure 20_K has extended to be the protrusive structure 20_K, K + 1. The content of the second regulation 85B is that in a case where the protrusive structure 20_K, K + 1 extends in one direction from the middle of the protrusive structure 20_K, it is determined that the protrusive structure 20_K has branched to be the protrusive structure 20_K, K + 1.

On the other hand, the content of the third regulation 85C is that in a case where the protrusive structure 20_K, K + 1 cross-shapedly intersect with the protrusive structure 20_K, it is determined that the protrusive structure 20_K, K + 1 is not the protrusive structure 20_K that extends from the same cell body 15.

The "same direction" of the first regulation 85A indicates, for example, a case where an angle formed by the protrusive structure 20_K and the protrusive structure 20_K, K + 1, where the protrusive structures are linearly approximated, is within a range of 160° to 200°. In a case where the angle is 180°, the protrusive structure 20_K and the protrusive structure 20_K, K + 1 are linearly connected.

It is also conceivable that the protrusive structure 20 is interrupted in some places due to noise. For this reason, in the first regulation 85A, it may be determined that the protrusive structure 20_K has extended in the case where the protrusive structure 20_K, K + 1 is present at a distance separated within a threshold value from the end point of the protrusive structure 20_K and extends in the same direction as the protrusive structure 20_K. Similarly, in the second regulation 85B, it may be determined that the protrusive structure 20_K has branched in the case where the protrusive structure 20_K, K + 1 is present at a distance separated within a threshold value from the middle of the protrusive structure 20_K and extends in one direction. The threshold value is set to, for example, 10 µm in terms of actual size. The method of specifying, for every nerve cell 11, the axon 16 and the dendrite 17 that extend from the cell body 15 is not limited to the method described above.

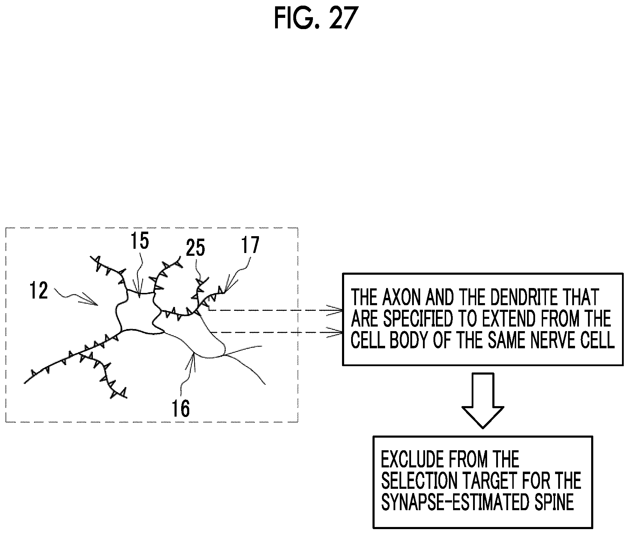

As conceptually illustrated in Fig. 27, the selection unit 81 excludes the protrusive structure 20 (the axon 16 and the dendrite 17), which has been specified to extend from the cell body 15 of the same nerve cell 11 in the specifying unit 80, from the selection target for the synapse-estimated spine 25S. Then, the selection unit 81 selects the synapse-estimated spine 25S.

In this manner, in the third embodiment, the specifying unit 80 specifies, for each nerve cell 11, the axon 16 and the dendrite 17 that extend from the cell body 15. Then, the axon 16 and the dendrite 17 that have been specified to extend from the cell body 15 of the same nerve cell 11 are excluded by the selection unit 81 from the selection target for the synapse-estimated spine 25S. As a result, it is possible to increase the probability that the synapse-estimated spine 25S actually constitutes the synapse 27.

After selecting the synapse-estimated spine 25S, the selection of the synapse-estimated spine 25S with respect to the protrusive structure 20, which has been specified to extend from the cell body 15 of the same nerve cell 11 in the specifying unit 80, may be canceled. In this case as well, eventually, the fact that the protrusive structure 20, which has been specified to extend from the cell body 15 of the same nerve cell 11 in the specifying unit 80, is excluded from the selection target for the synapse-estimated spine 25S does not change.

### [Fourth embodiment]

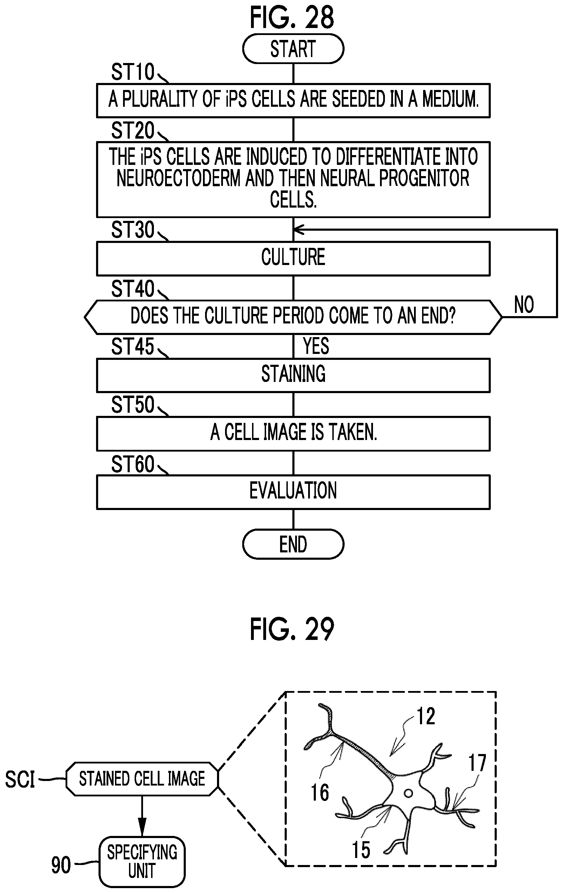

In the fourth embodiment illustrated in Fig. 28 and 29, a stained cell image SCI in which the nerve cell 11 in which at least any one of the axon 16 or the dendrite 17 is stained is shown is used as the cell image CI.

As illustrated in Fig. 28, in the present embodiment, the operator stains the nerve cells 11 before taking an image with the imaging device 12 (a step ST45). More specifically, the tau protein in the axon 16 is stained with a dye. Alternatively, the microtubule associated protein 2 (MAP2) protein in the dendrite 17 is stained with a dye.

As illustrated in Fig. 29, the acquisition unit 90 acquires the stained cell image SCI in which the nerve cell 11 (the nerve cell 11 in which the axon 16 is stained, in Fig. 29) in which at least any one of the axon 16 or the dendrite 17 is stained is shown. Since the subsequent processing is the same as the processing of each of the above embodiments, the description thereof will be omitted.

In this manner, in the fourth embodiment, the acquisition unit 90 acquires a stained cell image SCI in which the nerve cell 11 in which at least one of the axon 16 and the dendrite 17 is stained is shown. As a result, it is possible to easily specify the axon 16 and the dendrite 17 in the specifying units 47 and 80.

The timing at which the nerve cell 11 is evaluated in the cell evaluation device 10 is not limited to the timing after the end of the culture period illustrated in Fig. 3. The evaluation may be carried out in the middle of the culture period.

Various modifications may be made to the hardware configuration of the computer that constitutes the cell evaluation device 10. For example, the cell evaluation device 10 may be composed of a plurality of computers separated as hardware for the purpose of improving processing capacity and reliability. Specifically, the functions of the RW control unit 45 and the acquisition unit 46 and the functions of the specifying unit 47, the selection unit 48, and the display control unit 49 are distributed to two computers. In this case, the cell evaluation device 10 is composed of two computers.

The first specifying unit that specifies the axon 16, the second specifying unit that specifies the spine 25, the third specifying unit that specifies the dendrite 17, and the fourth specifying unit that specifies, for every nerve cell 11, the axon 16 and the dendrite 17 that extend from the cell body 15 may be provided separately.

In this manner, the hardware configuration of the computer of the cell evaluation device may be appropriately modified according to necessary performance such as processing capacity, security, and reliability. Further, as well as the hardware, the application program such as the operation program 40 may be duplicated or stored in a plurality of storage devices in a distributed manner for the purpose of ensuring security and reliability.

In each of the above embodiments, for example, as a hardware structure of processing units that execute various processing, such as the RW control unit 45, the acquisition units 46 and 90, the specifying units 47 and 80, the selection units 48 and 81, the display control unit 49, and the calculation unit 70, the various processors described below may be used. As described above, in addition to the CPU 32 that is a general-purpose processor that executes software (operation program 40) to function as various processing units, various processors include a programmable logic device (PLD) that is a processor of which a circuit configuration is changeable after manufacturing, such as a field programmable gate array (FPGA), a dedicated electrical circuit that is a processor having a circuit configuration specifically designed to execute a specific process, such as an application specific integrated circuit (ASIC), or the like.

One processing unit may be composed of one of these various processors or may be composed of a combination of two or more processors of the same type or different types (for example, a combination of a plurality of FPGAs and/or a combination of a CPU and an FPGA). Further, a plurality of processing units may be composed of one processor.

As an example in which the plurality of processing units is composed of one processor, first, as represented by a computer such as a client and a server, there is a configuration in which one processor is composed of a combination of one or more CPUs and software and the processor functions as a plurality of processing units. Secondly, as represented by a system on chip (SoC) or the like, there is a configuration in which a processor that realizes the functions of the entire system including a plurality of processing units by one integrated circuit (IC) chip is used. As described above, one or more of the above various processors are used to constitute the hardware structure of the various processing units.

Further, as a hardware structure of these various processors, more specifically, an electrical circuit (circuitry) in which circuit elements such as semiconductor elements are combined may be used.

From the above description, the disclosure described in Supplementary note 1 below can be understood.

### [Supplementary note 1]

A cell evaluation device comprising:
an acquisition processor that acquires a cell image in which a nerve cell having a cell body, an axon, and a dendrite is shown;
a first specification processor that specifies the axon in the cell image;
a second specification processor that specifies spines formed on the dendrite in the cell image;
a selection processor that selects a synapse-estimated spine, which is estimated to constitute a synapse with the axon and is close to the axon specified in the first specification processor, from the spines specified in the second specification processor; and
a display control processor that performs control to display the synapse-estimated spine in a display form different from that of other spines in the cell image.

The technique of the present disclosure may be appropriately combined with the above-described various embodiments and various modifications. In addition, it is needless to say that various configurations may be adopted within a scope without departing from the concept of the present disclosure and are not limited to each of the above embodiments. Further, the technique of the present disclosure extends to a storage medium that stores the program in a non-temporary manner, in addition to the program.

The above-described content and the above-illustrated content are detailed descriptions of portions related to the technique of the present disclosure, which are merely an example of the technique of the present disclosure. For example, the description of the above configurations, functions, operations, and effects is an example of the description of configurations, functions, operations, and effects of portions related to the technique of the present disclosure. Accordingly, within the scope without departing from the concept of the technique of the present disclosure, unnecessary portions may be removed, new elements may be added or replaced for the above-described content and the above-illustrated content. In addition, in order to avoid complication and facilitate understanding of the portions related to the technique of the present disclosure, in the above-described content and the above-illustrated content, description of common knowledge or the like that does not need special explanation in implementing the technique of the present disclosure is omitted.

In the present specification, "A and/or B" is synonymous with "at least one of A or B". That is, "A and/or B" may refer to only A, only B, or a combination of A and B. In addition, in the present specification, the same concept as "A and/or B" is also applied to a case where three or more matters are linked by "and/or".

All documents, patent applications, and technical standards disclosed in this specification are incorporated in this specification by reference in such a manner that the incorporation by reference of the individual document, patent application, and technical standard are handled to the same extent as in the specific and individual description thereof.

## Claims

1. A cell evaluation device comprising:
an acquisition unit that acquires a cell image in which a nerve cell having a cell body, an axon, and a dendrite is shown;
a first specifying unit that specifies the axon in the cell image;
a second specifying unit that specifies spines formed on the dendrite in the cell image;
a selection unit that selects a synapse-estimated spine, which is estimated to constitute a synapse with the axon and is close to the axon specified in the first specifying unit, from the spines specified in the second specifying unit; and
a display control unit that performs control to display the synapse-estimated spine in a display form different from that of other spines in the cell image.

2. The cell evaluation device according to claim 1,
wherein the selection unit selects the spine that is present at a distance of less than 1 µm in terms of actual size from the axon specified in the first specifying unit, as the synapse-estimated spine.

3. The cell evaluation device according to claim 1 or 2, further comprising an output control unit that performs control to output the number of the synapse-estimated spines.

4. The cell evaluation device according to claim 3,
wherein the output control unit performs control to output a proportion of the number of the synapse-estimated spines with respect to the total number of the spines specified in the second specifying unit.

5. The cell evaluation device according to claim 3 or 4, further comprising:
a third specifying unit that specifies the dendrite in the cell image,
wherein the output control unit performs control to output at least any one of the number of the synapse-estimated spines per unit length of the dendrite specified in the third specifying unit or the number of the synapse-estimated spines per unit area of the dendrite specified in the third specifying unit.

6. The cell evaluation device according to any one of claims 1 to 5, further comprising:
a fourth specifying unit that specifies, for every nerve cell, the axon and the dendrite that extend from the cell body,
wherein the selection unit excludes axons and dendrites, which have been specified to extend from a cell body of the same nerve cell in the fourth specifying unit, from a selection target for the synapse-estimated spine.

7. The cell evaluation device according to any one of claims 1 to 6,
wherein the acquisition unit acquires a stained cell image in which the nerve cell in which at least any one of the axon or the dendrite is stained is shown, as the cell image.

8. An operation method for a cell evaluation device, comprising:
an acquisition step of acquiring a cell image in which a nerve cell having a cell body, an axon, and a dendrite is shown;
a first specification step of specifying the axon in the cell image;
a second specification step of specifying spines formed on the dendrites in the cell image;
a selection step of selecting a synapse-estimated spine, which is estimated to constitute a synapse with the axon and is close to the axon specified in the first specification step, from the spines specified in the second specification step; and
a display control step of performing control to display the synapse-estimated spine in a display form different from that of other spines in the cell image.

9. A computer-readable storage medium storing an operation program for a cell evaluation device that causes a computer to function as:
an acquisition unit that acquires a cell image in which a nerve cell having a cell body, an axon, and a dendrite is shown;
a first specifying unit that specifies the axon in the cell image;
a second specifying unit that specifies spines formed on the dendrite in the cell image;
a selection unit that selects a synapse-estimated spine, which is estimated to constitute a synapse with the axon and is close to the axon specified in the first specifying unit, from the spines specified in the second specifying unit; and
a display control unit that performs control to display the synapse-estimated spine in a display form different from that of other spines in the cell image.
